(19) **Europäisches Patentamt European Patent Office Office européen des brevets**

(11) **EP 1 760 157 B1**

(12) **EUROPEAN PATENT SPECIFICATION**

(45) Date of publication and mention
of the grant of the patent:
**15.07.2009 Bulletin 2009/29**

(51) Int Cl.:
*C12P 23/00* *(2006.01)*

(21) Application number: **06254619.7**

(22) Date of filing: **05.09.2006**

(54) **Green algae having a high astaxanthin content and method for producing the same**

Grünalgen mit hohem Astaxanthingehalt und Verfahren zur deren Herstellung

Algues vertes à teneur elevée en astaxanthine, et procédé de préparation de celles-ci

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.09.2005 JP 2005258299**

(43) Date of publication of application:
**07.03.2007 Bulletin 2007/10**

(73) Proprietor: **Yamaha Hatsudoki Kabushiki Kaisha Iwata-shi, Shizuoka 438-8501 (JP)**

(72) Inventor: **Zhang, Kai**
**Iwata-shi,**
**Shizuoka 438-8501 (JP)**

(74) Representative: **Schlich, George William et al**
**Schlich & Co**
**34 New Road**
**Littlehampton**
**West Sussex BN17 5AT (GB)**

(56) References cited:
**EP-A1- 1 138 757        WO-A-94/23594**
**WO-A-97/28274          US-A1- 2004 091 524**

• **KANG C D ET AL: "Comparison of heterotrophic and photoautotrophic induction on astaxanthin production by Haematococcus pluvialis" APPLIED MICROBIOLOGY AND BIOTECHNOLOGY, vol. 68, no. 2, August 2005 (2005-08), pages 237-241, XP002411625 ISSN: 0175-7598**

• **HARKER M ET AL: "Autotrophic growth and carotenoid production of Haematococcus pluvialis in a 30 liter air-lift photobioreactor" JOURNAL OF FERMENTATION AND BIOENGINEERING, vol. 82, no. 2, 1996, pages 113-118, XP002334962 ISSN: 0922-338X**

• **FABREGAS J ET AL: "Interactions between irradiance and nutrient availability during astaxanthin accumulation and degradation in Haematococcus pluvialis." APPLIED MICROBIOLOGY AND BIOTECHNOLOGY, vol. 61, no. 5-6, June 2003 (2003-06), pages 545-551, XP002411626 ISSN: 0175-7598**

• **LABABPOUR A ET AL: "Effects of nutrient supply methods and illumination with blue light emitting diodes (LEDs) on astaxanthin production by Haematococcus pluvialis" JOURNAL OF BIOSCIENCE AND BIOENGINEERING, vol. 98, no. 6, December 2004 (2004-12), pages 452-456, XP002411627 ISSN: 1389-1723**

• **KATSUDA T ET AL: "Astaxanthin production by Haematococcus pluvialis under illumination with LEDs" ENZYME AND MICROBIAL TECHNOLOGY, vol. 35, no. 1, 6 July 2004 (2004-07-06), pages 81-86, XP002411628 ISSN: 0141-0229**

• **KOBAYASHI M ET AL: "Effects of light intensity, light quality, and illumination cycle on astaxanthin formation in a green alga, Haematococcus pluvialis" JOURNAL OF FERMENTATION AND BIOENGINEERING, vol. 74, no. 1, 1992, pages 61-63, XP002411629 ISSN: 0922-338X**

• **BOUSSIBA S ET AL: "Enhamcement and determination of astaxanthin accumulation in green alga Haematococcus pluvialis" METHODS IN ENZYMOLOGY, vol. 213, no. PART A, 1992, pages 386-391, XP000673890 ISSN: 0076-6879**

EP 1 760 157 B1

- **TJAHJONO A E ET AL: "Isolation of resistant mutants against carotenoid biosynthesis inhibitors for a green alga Haematococcus pluvialis, and their hybrid formation by protoplast fusion for breeding of higher astaxanthin producers" JOURNAL OF FERMENTATION AND BIOENGINEERING, vol. 77, no. 4, 1994, pages 352-357, XP002411630 ISSN: 0922-338X**
- **TJAHJONO A E ET AL: "Hyper-accumulation of astaxanthin in a green alga Haematococcus pluvialis at elevated temperatures" BIOTECHNOLOGY LETTERS, vol. 16, no. 2, 1994, pages 133-138, XP008072951 ISSN: 0141-5492**
- **KANG ET AL: "Astaxanthin biosynthesis from simultaneous N and P uptake by the green alga Haematococcus pluvialis in primary-treated wastewater" BIOCHEMICAL ENGINEERING JOURNAL, vol. 31, no. 3, 1 October 2006 (2006-10-01), pages 234-238, XP005668790 ISSN: 1369-703X**

- **KIM ET AL: "Enhanced production of astaxanthin by flashing light using Haematococcus pluvialis" ENZYME AND MICROBIAL TECHNOLOGY, vol. 39, no. 3, 3 July 2006 (2006-07-03), pages 414-419, XP005459375 ISSN: 0141-0229**
- **SUH ET AL: "A novel double-layered photobioreactor for simultaneous Haematococcus pluvialis cell growth and astaxanthin accumulation" JOURNAL OF BIOTECHNOLOGY, vol. 125, no. 4, 1 October 2006 (2006-10-01), pages 540-546, XP005648226 ISSN: 0168-1656**
- **YOSHIMURA ET AL: "Effective utilization of transmitted light for astaxanthin production by Haematococcus pluvialis" JOURNAL OF BIOSCIENCE AND BIOENGINEERING, vol. 102, no. 2, August 2006 (2006-08), pages 97-101, XP005685788 ISSN: 1389-1723**

Remarks:
    The file contains technical information submitted after the application was filed and not included in this specification

**Description**

BACKGROUND OF THE INVENTION

1. Field of the Invention

The present invention relates to efficient production of astaxanthin. More specifically, the present invention relates to a green alga having a high astaxanthin content and a method for producing the same.

2. Description of the Related Art

Astaxanthin, which is a carotenoid imparting a red color, is known to have a potent antioxidative effect. Thus, it is used as a pigment in food, a cosmetic, a health food product, a pharmaceutical, and the like. Some astaxanthins are chemically synthesized, and are also extracted from naturally occurring source. Naturally occurring astaxanthins are extracted, for example, from *Eucarida* such as euphausiids and *Pandalus borealis,* from *Phaffia* yeast, and from algae. However, astaxanthin cannot be produced efficiently from *Eucarida* such as euphausiids or from yeast because of their low astaxanthin content.

On the other hand, algae are encysted as a result of a change in the external environment and accumulate astaxanthin within algal cells. Thus, production of astaxanthin from algae has been investigated. Fabregas et al., J. Biotech., Vol. 89, pp. 65-71 (2001) describes a two-step culture in which vegetative cells of *Haematococcus* are obtained while exchanging 10 to 40% of a culture medium every day (i.e., fed-batch culture) and then batch culture is performed for an additional 15 days under irradiation with light. WO 89/01977 describes a process of producing astaxanthin by cultivating *Haematococcus* by varying the ratio of carbon and nitrogen, which are components of a culture medium, at a late stage of the cultivation. Moreover, Japanese Laid-Open Patent Publication No. 1-187082 describes a method for producing astaxanthin by cultivating algae in a culture medium supplemented with a metal salt. However, these documents do not describe the content of astaxanthin in algal cells.

Japanese Laid-Open Patent Publication No. 3-83577 discloses an astaxanthin content of 0.3 to 10 wt% in dry algal cells, but in an example in which algal cells were cultivated under nitrogen limitation and under culture conditions of 40000 lux, the astaxanthin content was about 2 wt%, and algal cells having an astaxanthin concentration of 10 wt% or more were not actually obtained. Furthermore, Japanese Laid-Open Patent Publication No. 2000-60532 discloses that *Haematococcus* cultivated in an outdoor culture pool contained 4.5 wt% of astaxanthin.

The astaxanthin content per algal cell also has been studied, and for example, Japanese Laid-Open Patent Publication No. 7-39389 and Tjahjono et al., BIOTECHNOLOGY LETTERS, Vol. 16, pp. 133-138 (1994) disclose that an amount of about 600 pg/cell of astaxanthin was produced through cultivation in the presence of iron ions and acetic acid at a culture temperature of 30°C. Japanese Laid-Open Patent Publication No. 2004-129504 discloses that it is possible to increase the amount of astaxanthin to a value of about 700 pg/cell or more. However, algae containing astaxanthin at such a high concentration were not actually obtained, and even the highest value in the examples described in Japanese Laid-Open Patent Publication No. 2004-129504 was only 156 pg/cell.

WO 2005/116238 discloses a method for efficiently producing xanthophyll by inoculating microalgae containing xanthophyll, e.g., encysted microalgae, into a nutrient medium to grow the microalgae as vegetative cells and further encysting the grown microalgae. In the examples in WO 2005/116238, the highest xanthophyll (astaxanthin) content in dry algal cells was 3.5 wt%.

Astaxanthin production by Haematococcus pluvialis is known from Kang et al: "Comparison of heterotrophic and photoautotrophic induction on astaxanthin production by Haematococcus pluvialis" APPLIED MICROBIOLOGY AND BIOTECHNOLOGY, vol. 68, no. 2, August 2005, pages 237-241, Lababpour et al: "Effects of nutrient supply methods and illumination with blue light emitting diodes (LEDs) on astaxanthin production by Haematococcus pluvialis" JOURNAL OF BIOSCIENCE AND BIOENGINEERING, vol. 98, no. 6, December 2004, pages 452-456, and Katsuda at al: "Astaxanthin production by Haematococcus pluvialis under illumination with LEDs" ENZYME AND MICROBIAL TECHNOLOGY, vol. 35, no. 1, 6 July 2004, pages 81-86. Kang et al discloses a maximum light intensity of 200 $\mu$mol-photon/m$^2$/s and an astaxanthin content in cells of 7.7% of biomass. Both Lababpour et al and Katsuda et al. disclose maximum light intensities of 12 $\mu$mol-photo/m$^2$/s.

For the purpose of efficiently producing astaxanthin, there has been a demand for algae containing astaxanthin at a higher concentration.

SUMMARY OF THE INVENTION

It is an object of the present invention to provide green algae having a high astaxanthin concentration in cells.

The present invention provides a method for producing a green alga, the method comprising cultivating an encysted green alga that can produce astaxanthin in a nutrient medium with a supply of carbon dioxide under irradiation with light at a photosynthetically active photon flux input of 25000 $\mu$mol-photon/m$^3$/s or more.

The present invention also provides a green alga that can produce astaxanthin containing astaxanthin in an amount of 700 pg/cell or more.

The present invention also provides a green alga containing astaxanthin at a concentration of 1.2 wt% or more per wet algal cell.

Furthermore, the present invention provides a method for producing a dry product of a green alga, the method comprising:

cultivating an encysted green alga that can produce astaxanthin in a nutrient medium with a supply of carbon dioxide under irradiation with light at a photosynthetically active photon flux input of 25000 $\mu$mol-photon/m$^3$/s or more; and drying the resultant green alga.

The present invention further provides a dry product of a green alga that can produce astaxanthin, which contains astaxanthin at a concentration of 5 wt% or more and contains astaxanthin in an amount of 700 pg/cell or more.

The present invention further provides a method for producing a culture suspension of a green alga containing astaxanthin at a concentration of 150 mg or more per 1 L of the culture suspension, the method comprising:

cultivating an encysted green alga that can produce astaxanthin in a nutrient medium with a supply of carbon dioxide under irradiation with light at a photosynthetically active photon flux input of 25000 $\mu$mol-photon/m$^3$/s or more.

The present invention also provides a culture suspension of a green alga containing astaxanthin at a concentration of 150 mg or more per 1 L of the culture suspension.

The present invention further provides a method for producing astaxanthin, the method comprising:

cultivating an encysted green alga that can produce astaxanthin in a nutrient medium with a supply of carbon dioxide under irradiation with light at a photosynthetically active photon flux input of 25000 $\mu$mol-photon/m$^3$/s or more; drying the resultant green alga; and extracting astaxanthin from the resultant dry product of the green alga.

In one embodiment, in the above method, the nutrient medium is an autotrophic medium.

In one embodiment, in the above method, the photosynthetically active photon flux input is not more than 750000 $\mu$mol-photon/m$^3$/s.

In one embodiment, the green alga is a unicellular alga belonging to the genus *Haematococcus,* preferably *Haematococcus pluvialis.*

According to the present invention, a green alga having an astaxanthin content per cell of 700 pg (700 pg/cell) or more is provided. Since this green alga has a high astaxanthin content per cell, the production efficiency of astaxanthin is improved more than ever before, by using this green alga.

BRIEF DESCRIPTION OF THE DRAWINGS

Fig. 1 presents a graph showing the change in astaxanthin content per cell over time.
Fig. 2 presents a graph showing the change in astaxanthin production rate per cell over time.
Fig. 3 presents a graph showing the change in astaxanthin content per dry algal cell over time.
Fig. 4 presents a graph showing the change in astaxanthin concentration in a culture suspension over time.
Fig. 5 presents a graph showing the change in astaxanthin production rate per volume of culture suspension over time.
Fig. 6 presents a graph showing the change in dry weight of solids per volume of culture suspension over time.

DETAILED DESCRIPTION OF THE INVENTION

The green algae of the present invention contain astaxanthin in an amount of 700 pg/cell or more, preferably 1000 pg/cell or more. Such green algae can be obtained by inducing encystment under culture conditions of irradiation with light. Preferably, it can be obtained by inoculating encysted green algae into a nutrient medium, and culturing the algae while bubbling air into the culture medium and under irradiation with intense light. By such a culture method, a dry product of green algae having an astaxanthin content of 5 wt% per dry algal cell (50 mg/g dry algal cell) or more, preferably 6 wt% of a dry algal cell (60 mg/g dry algal cell) or more, can be obtained. Furthermore, the amount of astaxanthin per volume of culture suspension also reaches a value of 150 mg/L or more, preferably 200 mg/L or more, more preferably 250 mg/L or more.

Hereinafter, the green algae containing astaxanthin in an amount of 700 pg/cell or more; the dry product of the green algae containing astaxanthin at a concentration of 5 wt% (50 mg/g dry algal cell) or more; and the culture suspension of the green algae which contains astaxanthin at a concentration of 150 mg/L culture suspension will be described.

The method of the present invention is characterized in that green algae containing astaxanthin, preferably encysted green algae, are inoculated into a growth medium to grow the green algae, and further encysted under irradiation with light. When green algae containing astaxanthin, preferably encysted green algae in which a large amount of astaxanthin has been accumulated, are inoculated into a growth medium to grow the green algae, the encysted green algae release zoospores containing astaxanthin. These zoospores become vegetative cells while maintaining astaxanthin. Then, by further encysting these green algae (vegetative cells) containing astaxanthin, additional astaxanthin is newly produced and accumulated in the green algae. Therefore, the green algae encysted by the method of the present invention have a high astaxanthin content because they contain the newly produced astaxanthin in addition to the astaxanthin originally present in the green algae.

Green Algae

There is no particular limitation on the green algae used in the present invention, as long as the green algae can produce astaxanthin. For example, unicellular algae belonging to the genus *Haematococcus* are preferably used. Examples of the green algae include *Haematococcus pluvialis* (*H. pluvialis*), *Haematococcus lacustris* (*H. lacustris*), *Haematococcus capensis* (*H. capensis*), *Haematococcus droebakensi* (*H. droebakensi*), and *Haematococcus zimbabwiensis* (*H. zimbabwiensis*).

Examples of *Haematococcus pluvialis* (*H. pluvialis*) include the NIES144 strain deposited in the Independent Administrative Institution National Institute for Environmental Studies, the UTEX2505 strain deposited in the Culture Collection of Algae at the University of Texas, U.S.A., and the K0084 strain deposited in the Scandinavian Culture Center for Algae and Protozoa, Botanical Institute, at the University of Copenhagen, Denmark.

Examples of *Haematococcus lacustris* (*H. lacustris*) include the ATCC30402 and ATCC30453 strains deposited in ATCC, the IAM C-392, IAM C-393, IAM C-394, and IAM C-339 strains deposited in the Institute of Applied Microbiology, University of Tokyo, or the UTEX16 and UTEX294 strains.

Examples of *Haematococcus capensis* (*H. capensis*) include the UTEX LB1023 strain.

Examples of *Haematococcus droebakensi* (*H. droebakensi*) include the UTEX55 strain.

Examples of *Haematococcus zimbabwiensis* (*H. zimbabwiensis*) include the UTEX LB1758 strain.

Among these, *Haematococcus pluvialis* is preferably used.

Encystment

In the present invention, green algae described above that contain astaxanthin are used. When green algae are subjected to stresses from the environment, such as nutrient deprivation or the presence of oxides, the green algae accumulate astaxanthin within the cells and become resting spores. The shift to this resting state is referred to as encystment. In this specification, encystment refers to any state from the beginning of the resting state where accumulation of astaxanthin starts, to the completely encysted state where the cells become resting spores. In order to increase the astaxanthin content, it is preferable to use green algae in which encystment has progressed as far as possible and which has accumulated a large amount of astaxanthin. It should be noted that "cultivating encysted green algae" as used herein also includes the process of inoculating green algae containing astaxanthin that has been grown in a nutrient medium, after the algae has reached the encysted state. In the present specification, "green algae" are also intended to include encysted green algae.

Medium

There is no particular limitation on the medium used to cultivate the green algae. Generally, a medium is used that contains nitrogen, inorganic salts of trace metal (e.g., phosphorous, potassium, magnesium, and iron), vitamins (e.g., thiamine), and the like, which are essential to growth. For example, media such as the VT medium, C medium, MC medium, MBM medium, and MDM medium (see Sorui Kenkyuho, ed. by Mitsuo Chihara and Kazutoshi Nishizawa, Kyoritsu Shuppan (1979)), the OHM medium (see Fabregas et al., J. Biotech., Vol. 89, pp. 65-71 (2001)), the BG-11 medium, and modifications thereof may be used. In the present invention, it is preferable to use an autotrophic medium that is substantially free from organic carbon source so that contamination by bacteria can be prevented.

These media may be selected depending on their purposes, such as growth, or encystment. For example, for growth of the green algae, a medium having a large amount of components serving as a nitrogen source is used (rich medium: containing at least 0.15 g/L expressed in terms of nitrogen). For encystment, a medium having a small amount of components serving as a nitrogen source is used (encystment medium: containing less than 0.02 g/L expressed in terms of nitrogen). Alternatively, a medium containing a nitrogen source at an intermediate concentration between these media may be used (low nutrient medium: containing at least 0.02 g/L and less than 0.15 g/L expressed in terms of nitrogen).

The nitrogen source concentration, phosphorous concentration, and other properties of the medium can be determined depending on the amount of the green algae to be inoculated. For example, when a green algae count in the order of $10^5$ is inoculated in a low nutrient medium, the green algae would grow to a certain extent, but the growth may stop soon because the amount of the nitrogen source is too small. Such a low nutrient medium is suitable for performing growth and encystment continuously in a single step (in a batch manner), as described later. Furthermore, by adjusting the N/P mole ratio to value from 10 through 30, preferably 15 through 25, the green alga can be encysted.

In the case where the green algae count for inoculation is even higher, the rich medium can be employed to perform the above-described cultivation.

In this manner, the composition of the medium can be determined in consideration of various conditions. It should be noted that the medium preferably used in the present invention, i.e., an autotrophic medium, is nearly free from an organic carbon source such as acetic acid or glucose, so that contamination by bacteria hardly occurs even in long-term cultivation.

Culture Apparatus

There is no particular limitation on the apparatus for cultivating the green algae, as long as the apparatus is capable of supplying carbon dioxide and irradiating a culture suspension with light. For example, in the case of a small-scale culture, a flat culture flask may be preferably used. In the case of a large-scale culture, a culture tank that is constituted by a transparent plate made of glass, plastic, or the like and that is equipped with an irradiation apparatus and an agitator, if necessary, may be used. Examples of such a culture tank include a plate culture tank, a tube-type culture tank, an airdome-type culture tank, and a hollow cylinder-type culture tank. In any case, a sealed container is preferably used.

Culture Conditions

There is no particular limitation on the culture conditions, and a temperature, a pH, and the like as generally employed for cultivation of green algae can be used. The green algae are cultivated at, for example, 15 to 35°C, and preferably 20 to 25°C. It is preferable that the pH is maintained at 6 to 8 throughout the cultivation period. Carbon dioxide is supplied by bubbling a gas containing carbon dioxide at a concentration of 1 to 3 v/v% at a rate of 0.2 to 2 wm, for example. When a plate culture tank is used, the culture suspension is stirred by supplying carbon dioxide, so that the green algae can be uniformly irradiated with light.

Light Irradiation

In the present invention, the green algae are cultivated by irradiating the green algae with light so that the photosynthetically active photon flux input is about 25000 μmol-photon/m$^3$/s (hereinafter, this unit is abbreviated as μmol-p/m$^3$/s). The photosynthetically active photon flux input is preferably 30000 μmol-p/m$^3$/s or more, more preferably 40000 μmol-p/m$^3$/s or more, in order to increase the production of astaxanthin. Moreover, since cell growth is stopped when photosynthesis is inhibited by intense light, the photosynthetically active photon flux input is preferably not more than 750000 μmol-p/m$^3$/s. The production of astaxanthin is significantly increased by performing irradiation with light at such a photosynthetically active photon flux input throughout the entire cultivation process from the start of cultivation to encystment.

The photosynthetically active photon flux input can be obtained by first measuring the photosynthetically active photon flux density (PPFD). The PPFD can be obtained by placing a flat-surface photon sensor LI-190 (LICOR Inc., Lincoln, USA) at several points in the culture apparatus, performing irradiation with light to measure the PPFD at each of the points, and averaging the values obtained. When there is a plurality of light sources, the PPFD is the total of each PPFD received from the respective light sources. When the apparatus is constituted by a transparent plate such as glass or an acrylic resin, a light source may be positioned by measuring the PPFD passing through the transparent plate, and then determining the intensity of the light source or the distance of the light source required for obtaining a predetermined PPFD. When a culture tank constituted by two transparent flat plates is used and irradiation with light is performed from both sides of the culture tank, the PPFD is obtained by adding up the PPFD values from the respective sides.

The photosynthetically active photon flux input can be calculated using the following equation:

$$\text{Photosynthetically active photon flux input} = \frac{\text{PPFD} (\mu \text{ mol}/\text{m}^2/\text{s}) \times \text{ Light receiving area } (\text{m}^2)}{\text{Volume of alga culture } (\text{m}^3)}$$

Culture Method

Cultivation is performed under irradiation with light by selecting appropriately and combining the above-described medium, culture apparatus, culture conditions, and the like. There are two culture methods. One method is a single-step culture in which encysted green algae are grown and encysted continuously in the same medium. The other method is a two-step culture in which the medium for growing encysted green algae and the medium for encysting the grown green algae are different from each other, and growth and encystment are performed separately.

Single-Step Culture

The single-step culture is a method of cultivating the encysted green algae continuously without changing the medium during the period from inoculation of the encysted green algae into a nutrient medium to the end of the cultivation. In other words, growth and encystment of the green algae are performed with a predetermined medium in the same culture tank. In this single-step culture, once the green algae have grown, the green algae are shifted to an encysted state smoothly under at least one of the following stresses: nutrient starvation stress due to consumption of the nutrients in the medium, stress due to irradiation with light. The encysted green algae obtained by this single-step culture may be further used for a subsequent single-step culture or for inoculation into a medium for use in cultivation of the two-step culture described later.

When a green alga containing astaxanthin, preferably an encysted green alga, is inoculated into the nutrient medium, the green alga releases $2^n$ (n = 1 to 4) zoospores containing astaxanthin. These zoospores become vegetative cells which still contain astaxanthin, so that the number of vegetative cells containing astaxanthin increases (i.e., the green algae can grow). By encysting vegetative cells containing astaxanthin, astaxanthin is newly accumulated in addition to the astaxanthin originally contained in the green algae, and thus the astaxanthin content in the cells is increased even more.

It is believed that when the vegetative cells continue to grow, the astaxanthin concentration in the vegetative cells eventually decreases, and therefore, it is preferable to stop growth at a point where some astaxanthin remains in the cells.

In order to stop growth of the green alga at the point where the vegetative cells have grown to a certain extent, it is preferable that the medium be designed to cause nutrient-starvation. For this purpose, in the single-step culture, a medium having a relatively low nitrogen source concentration, e.g., the low nutrient medium described above, is preferably used. When a large amount of encysted cells is inoculated, a medium having a high nitrogen source concentration, e.g., the rich medium described above, may be used.

It should be noted that when the green algae do not grow sufficiently in the low nutrient medium, a rich medium or a low nutrient medium may be supplemented so as to grow the green algae to a certain extent.

Moreover, in the case where a low nutrient medium is used, if the N/P mole ratio is adjusted to value between 10 and 30, preferably between 15 and 25, then the green algae can be encysted smoothly after growth.

The single-step culture not only has advantages such as that process control can be performed easily, contamination by bacteria can be prevented because transfer to another culture tank is not necessary, and the method can be performed with a single culture tank, but also that it is possible to obtain readily green algae containing astaxanthin at high concentration.

Two-Step Culture

The two-step culture is a culture method in which encysted green algae are grown in a nutrient medium, and then encysted by changing the nutrient medium to an encystment medium. In other words, the two-step culture includes a first step in which encysted green algae are first inoculated into a nutrient medium, preferably a rich medium, to grow the green algae, and a second step in which the green algae are collected and transferred to an encystment medium that is nearly free from a nitrogen source, and then encysted.

Since it is necessary to finish growth of the green algae in the first step while astaxanthin remains in the vegetative cells, cultivation in the nutrient medium is performed for a short period of time. When the cultivation is performed using a rich medium at the start of the cultivation, the growth rate of the vegetative cells is higher than the growth rate when a low nutrient medium is used, and therefore it is preferable to use a rich medium. After growth, the green algae are collected and transferred to an encystment medium, and encysted in the second step.

The first step and the second step may be performed independently in a batch manner using separate culture tanks. It is also possible to wash and collect the grown green algae at the end of the first step, place the green algae back in the same culture tank, and then perform the second step.

With this two-step culture, green algae having high astaxanthin content can also be obtained. This two-step culture has an advantage in that the growth step can be finished in a shorter period of time than with the single-step culture; however, the operation of transferring the grown green algae is required in the two-step process.

It is also possible to use a portion of the obtained encysted green algae for collecting astaxanthin and a portion of the remainder for another inoculation into a nutrient medium.

Measurement of the Amount of Astaxanthin

By the above-described cultivation, a culture suspension containing astaxanthin (in terms of free or unesterified form) at a concentration of 150 mg or more per 1 L of the culture suspension, preferably 150 to 250 mg/L, more preferably 200 to 250 mg/L, can be obtained. The amount of astaxanthin per volume of culture suspension can be obtained by collecting a predetermined volume of the culture suspension and measuring the amount of astaxanthin contained in the collected culture suspension.

The amount of astaxanthin (in terms of free or unesterified form) in a sample (culture suspension) can be measured by the following method, for example. First, a certain amount of the sample is collected, washed, and taken into a microtube designed for exclusive use with a bead beater. After adding zirconia beads to the tube, acetone is added thereto, and the sample is beaten with the bead beater. After the beating step, the sample is separated into a supernatant and a precipitate by centrifugation, and the supernatant (i.e., an acetone fraction) is collected. Acetone is added again to the precipitate, and the same operation as described above is repeated until the color of the precipitate becomes almost completely white. The collected acetone fractions are combined and diluted 100-fold with dimethyl sulfoxide (DMSO), and the absorbance at 492 nm ($A_{492}$) and the absorbance at 750 nm ($A_{750}$) are measured. The astaxanthin concentration in the collected acetone fractions (sample) can be calculated using the following equation, and the amount of astaxanthin in the culture suspension can be obtained from this calculated value:

$$\text{Astaxanthin concentration in sample } (\mu g/mL) = 4.5 \times 100 \times (A_{492} - A_{750})$$

Wet Algal Cells Containing Astaxanthin

By performing cultivation under the above-described conditions, green algae containing astaxanthin (in terms of free or unesterified form) in an amount of 700 pg/cell or more, preferably 800 pg/cell or more, more preferably 900 pg/cell or more, even more preferably 1000 pg/cell or more, can be obtained. According to the method of the present invention, green algae containing astaxanthin in an amount of 1100 pg/cell or more (e.g., 1200 pg/cell) can be obtained.

"Wet algal cells" as used herein refers to algal cells in which moisture is not removed from the algal cells and the inside of the algal cells is filled with fluid. An example thereof is green algae that are present in a culture suspension.

The amount of astaxanthin per cell can be obtained by collecting a predetermined amount of green algae and measuring the number of cells in the collected green algae and the amount of astaxanthin extracted from the cells. The number of cells can be measured using an apparatus for the measurement of particle size distribution (SYSMEX FPI-3000), for example.

Dry Product of Green Algae Containing Astaxanthin

The wet algal cells containing astaxanthin described above are dried by a drying means commonly used by those skilled in the art (e.g., drum drying, hot air drying, spray drying, or freeze-drying), so that a dry product of green algae can be obtained.

The obtained dry product of the green algae contains astaxanthin (in terms of free or unesterified form) at a concentration of 5 wt% or more. Preferably, it contains astaxanthin at a concentration of 5 to 8 wt%, more preferably 6 to 7 wt%. It should be noted that the moisture content in the "dry product" as used herein is 7 wt% or less, preferably 5 wt%, more preferably about 2 wt%.

Method for Producing Astaxanthin

Astaxanthin can be produced by extracting astaxanthin from the obtained dry product of the green algae and collecting the extracted astaxanthin. There is no particular limitation on the method of extracting and collecting astaxanthin, and a method commonly used by those skilled in the art can be employed. For example, the dry product of the green algae can be mechanically destroyed, and then astaxanthin is extracted therefrom. The extraction methods include chemical extraction using an organic solvent, such as chloroform, hexane, acetone, methanol, or ethanol, or an edible oil or fat, and physical extraction, for example, by squeezing the dry product of the green algae. Alternatively, supercritical extraction may be employed for extraction and collection. After the extraction, astaxanthin can be purified by such a method as concentration and crystallization, or fractionation using a synthetic resin (e.g., styrene-divinylbenzene copolymer).

Examples

Hereinafter, the present invention will be described by means of examples in which *Haematococcus pluvialis* K0084 strain is used.

In the examples, the amount of astaxanthin and the number of cells were measured according to the above-described methods.

The method for measuring the dry weight of green algae is as follows. First, a predetermined volume of a culture suspension was collected and filtered on a GC50 glass fiber filter (made by Advantec Toyo Kaisha, Ltd.) under reduced pressure, and then washed twice with 5 mL of an aqueous solution of hydrochloric acid at pH 4 to dissolve inorganic salts. Then, the filter to which the green algae were adsorbed was dried in a thermostatic drier at 105°C for 3 hours and cooled in a vacuum desiccator for one hour to room temperature, and then the weight of the dried filter was measured. The weight of the GC50 glass fiber filter was preliminarily measured by drying the filter in the thermostatic drier at 105°C for one hour. The dry weight of the green algae was obtained by subtracting the dry weight of the filter that was preliminarily measured from the weight of the dried filter to which the green algae were adsorbed. Moreover, the amount of astaxanthin per dry weight was calculated from the measured value of the amount of astaxanthin in the predetermined volume of the culture suspension.

Example 1

Preparation of Encysted Green Algae

*Haematococcus pluvialis* K0084 strains were inoculated into a medium (low nutrient medium) containing the components shown in Table 1 below.

Table 1

| Components | g/L |
|---|---|
| $KNO_3$ | 0.41 |
| $K_2HPO_4$ | 0.04 |
| $MgSO_4 \cdot 7H_2O$ | 0.075 |
| $CaCl_2 \cdot 2H_2O$ | 0.036 |
| Citric acid (anhydrous) | 0.006 |
| Ammonium iron (III) citrate | 0.006 |
| EDTA$\cdot$2Na | 0.001 |
| $Na_2CO_3$ | 0.02 |
| $CuSO_4 \cdot 5H_2O$ | 0.00286 |
| $H_3BO_4$ | 0.00181 |
| $MnCl_2 \cdot 4H_2O$ | 0.00022 |
| $ZnSO_4 \cdot 7H_2O$ | 0.00008 |
| $Na_2MoO_4$ | 0.000021 |
| $Co(NO_3)_2 \cdot 6H_2O$ | 0.000000494 |

More specifically, 1 L of the low nutrient medium was placed in a 1.5 L flat culture flask, and the encysted K0084 strains were inoculated into the medium. The K0084 strains were cultivated for 7 days at 25°C under light irradiation at a photosynthetically active photon flux input of 25000 $\mu$mol-p/m$^3$/s using a white fluorescent lamp, while bubbling a gas containing 3 vol% of $CO_2$ into the medium at a rate of 0.5 Umin (i.e., at an aeration rate of 0.5 vvm). After 7 days, the encysted K0084 strains were collected, and adjusted so that the concentration of the encysted K0084 strains in the low nutrient medium was $1.5 \times 10^6$ cells/ml.

Main Culture

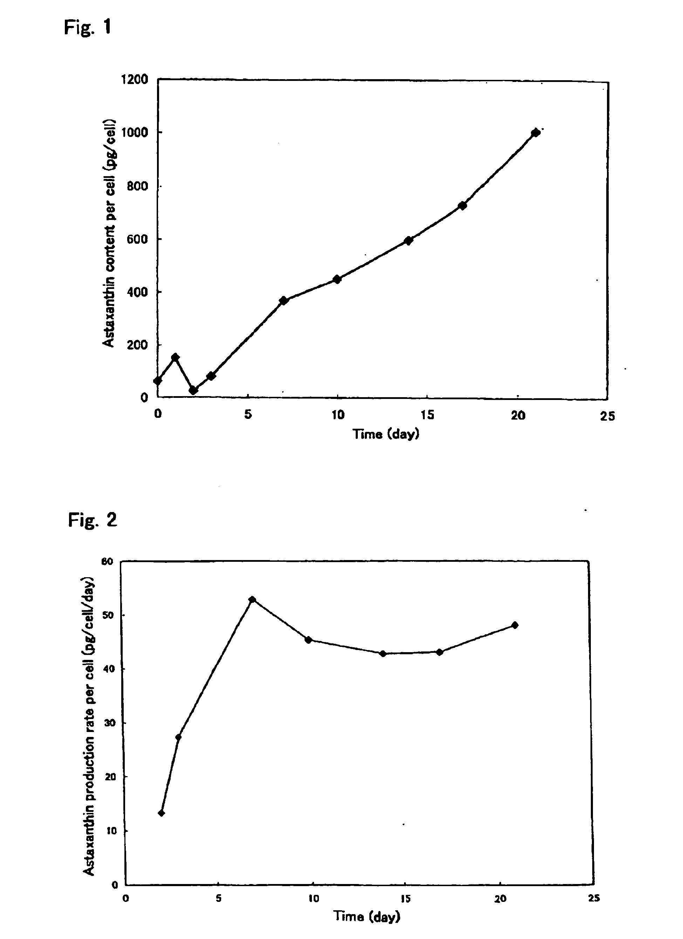

First, 9 L of the low nutrient medium were placed in a flat culture tank in which acrylic transparent plates are opposite to each other so that the distance between the inner walls of the culture tank was 3 cm, and 1 L of the above-described encysted K0084 strains were inoculated into the medium so that the concentration was $1.5 \times 10^5$ cells/ml to start cultivation. The encysted K0084 strains were cultivated for 21 days at 25°C under irradiation with light at a photosynthetically active photon flux input of 25000 $\mu$mol-p/m$^3$/s using six white fluorescent lamps provided on each side of the flat culture tank, while bubbling a gas containing 3 vol% of $CO_2$ into the medium at a rate of 5 Umin (i.e., at an aeration rate of 0.5 vvm). The astaxanthin content in the culture suspension, the number of cells, and the dry weight of algal cells collected from the culture suspension were measured regularly by the above-described methods. The results are shown in Table 2 and Figs. 1 to 6.

The number of living cells increased by a factor of about 4 to a value of $6.5 \times 10^5$ cells/ml on day 3, but after that day, it decreased slowly (not shown). The astaxanthin content per living cell reached a value of 600 pg/cell on day 14, 800 pg/cell or more on day 18, and 900 pg/cell or more on day 19, and furthermore, it reached a value of 1000 pg/cell on day 21, as shown in Fig. 1. Moreover, as shown in Fig. 2, the astaxanthin production rate per cell was the highest around day 7 of the cultivation and was about 54 pg a day per cell. Thereafter, astaxanthin was produced at a rate of about 45 pg a day per cell.

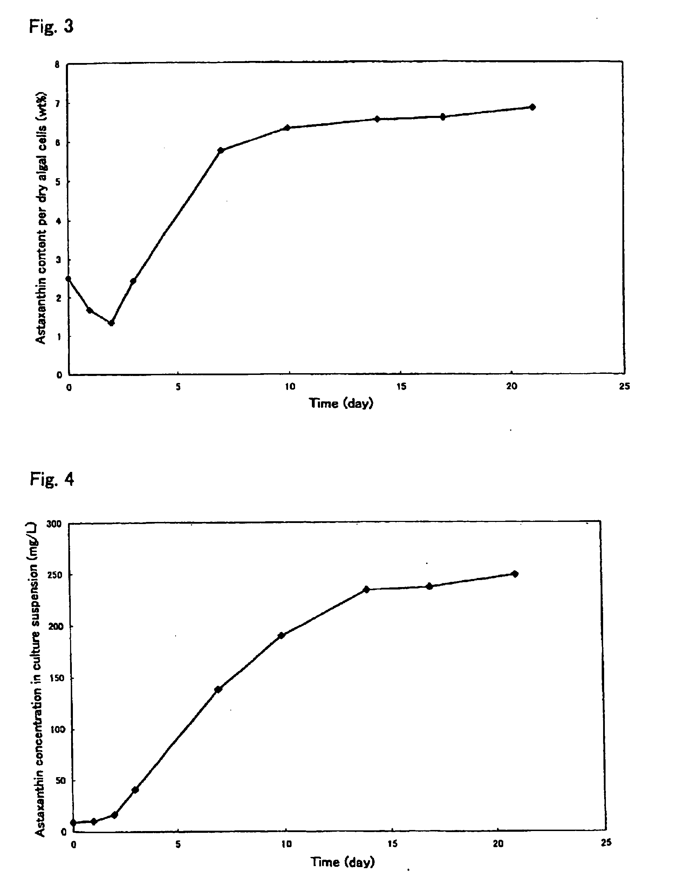

As shown in Fig. 3, the astaxanthin content per dry algal cells decreased until day 3 by which time the cells had grown vegetatively, but after the start of encystment, it increased rapidly and reached a value of 6 wt% (60 mg/g dry algal cells) on day 7. When the cultivation was further continued, the amount of astaxanthin per algal cells kept increasing gradually and reached a value of 6.8 wt% (68 mg/g dry algal cells) on day 21.

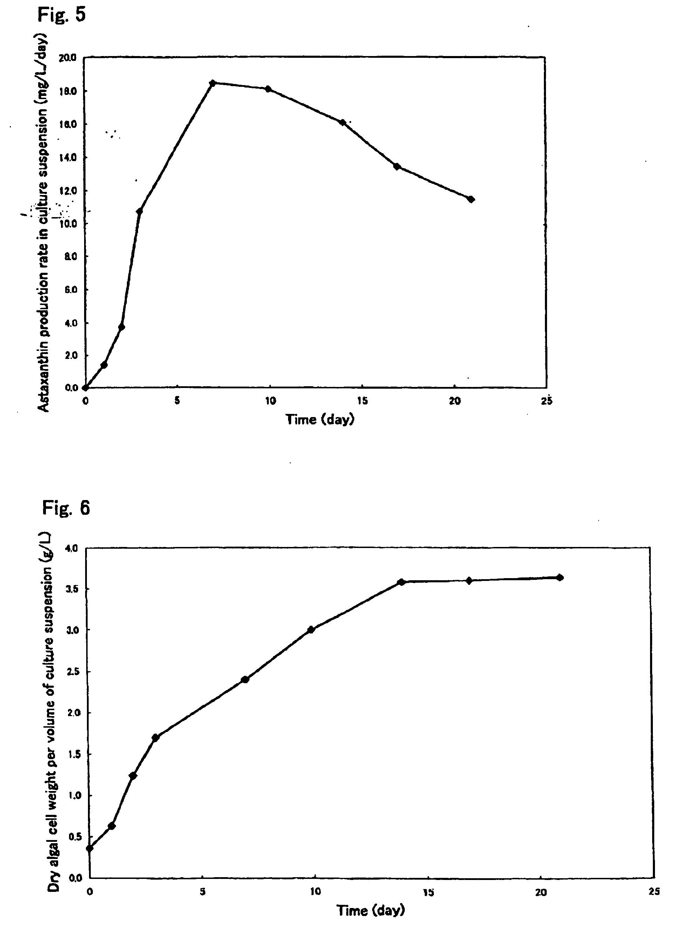

Fig. 4 shows the change in astaxanthin concentration in the culture suspension over time. On day 7 of the cultivation, the concentration was as high as 150 mg per 1 L of the culture suspension, and the concentration became 200 mg/L after day 10 and was 250 mg/L on day 21. Moreover, as shown in Fig. 5, the daily astaxanthin production rate per 1 ml of the culture suspension was the highest around day 7 of the cultivation and was about 19 mg a day per culture suspension. Thereafter, the astaxanthin production rate decreased slightly.

As shown in Fig. 6, the weight of solids in the culture suspension increased as the cultivation proceeded, reached a peak on day 13, and after that day, remained nearly unchanged. Giving consideration to the fact that the number of living cells decreased slowly, it is apparent that the increase in the solid weight results from an increase in the weight of the cells due to encystment.

Comparative Example 1

Cultivation was performed in the same manner as in Example 1, except that irradiation with light was performed at a photosynthetically active photon flux input of 12000 $\mu$mol-p/m$^3$/s, and the astaxanthin concentration per wet algal cells, the astaxanthin concentration in dry algal cells, and the amount of astaxanthin per 1 L of the culture suspension were measured on day 21. The results are shown in Table 2.

Comparative Example 2

Cultivation was performed in the same manner as in Example 1, except that irradiation with light was performed so that the photosynthetically active photon flux input was 8000 $\mu$mol-p/m$^3$/s, which is the value of the photosynthetically active photon flux input described in Tjahjono et al., BIOTECHNOLOGY LETTERS, Vol. 16, pp. 133-138 (1994) and is employed in common cultivation processes of *Haematococcus.* The astaxanthin concentration per wet algal cells, the astaxanthin concentration in dry algal cells, and the amount of astaxanthin per 1 L of the culture suspension were measured on day 21. The results are shown in Table 2.

Table 2

| | Photosynthetically active photon flux input [1] flux | Astaxant hin concentration on day 21 | | | |
|---|---|---|---|---|---|
| | | pg/cell | % (w/ w per wet algal cells) | % (w/ w per dry algal cells) | mg/L culture suspension |
| Example 1 | 25000 | 1000 | 2.7 | 6.8 | 250 |
| Comparative Example 1 | 12000 | 650 | 1.8 | 4.7 | 140 |
| Comparative Example 2 | 8000 | 250 | 1.3 | 3.1 | 120 |
| [1]:$\mu$mol-p/m$^3$/s | | | | | |

Since the algae of the present invention contains astaxanthin at a higher concentration than algae that are conventionally known, it is possible to significantly improve the production efficiency of astaxanthin, which is used in the fields of antioxidants, food products, pharmaceuticals, and cosmetics and other fields. Therefore, the algae of the present invention are very useful in a method for efficiently producing astaxanthin.

**Claims**

1. A method for producing a green alga, comprising cultivating an encysted green alga that can produce astaxanthin in a nutrient medium with a supply of carbon dioxide under irradiation with light at a photosynthetically active photon flux input of 25000 $\mu$mol-photon/m$^3$/s or more.

2. The method of claim 1, wherein the nutrient medium is an autotrophic medium.

3. The method of claim 1 or 2, wherein the photosynthetically active photon flux input is not more than 750000 $\mu$mol-photon/m$^3$/s.

4. The method of any of claims 1-3, wherein the green alga contains astaxanthin at a concentration of 1.2 wt% or more per wet algal cell.

5. The method of any of claims 1-4, wherein the supply of carbon dioxide is provided by bubbling a gas containing carbon dioxide at a concentration of 1 to 3 v/v% at a rate of 0.2 to 2 v/v/min.

6. A green alga that can produce astaxanthin, which contains astaxanthin in an amount of 700 pg/cell or more.

7. The green alga of claim 6, which contains astaxanthin at a concentration of 1.2 wt% or more per wet algal cell.

8. The green alga of claim 6 or 7, which is a unicellular alga belonging to the genus *Haematococcus*.

9. The green alga of claim 8, which is *Haematococcus pluvialis*.

10. A method for producing a dry product of a green alga, comprising:

    cultivating an encysted green alga that can produce astaxanthin in a nutrient medium with a supply of carbon dioxide under irradiation with light at a photosynthetically active photon flux input of 25000 $\mu$mol-photon/m$^3$/s or more; and
    drying the resultant green alga.

11. The method of claim 10, wherein the nutrient medium is an autotrophic medium.

12. The method of claim 10 or 11, wherein the photosynthetically active photon flux input is not more than 750000 $\mu$mol-photon/m$^3$/s.

**13.** The method of claim 10, 11 or 12, wherein the dry product contains astaxanthin at a concentration of 5 wt% or more.

**14.** The method of any of claims 10-13, wherein the supply of carbon dioxide is provided by bubbling a gas containing carbon dioxide at a concentration of 1 to 3 v/v% at a rate of 0.2 to 2 v/v/min.

**15.** A dry product of a green alga that can produce astaxanthin, which contains astaxanthin at a concentration of 5 wt% or more and contains astaxanthin in an amount of 700 pg/cell or more.

**16.** The dry product of a green alga of claim 15, wherein the green alga is a unicellular alga belonging to the genus *Haematococcus*.

**17.** The dry product of a green alga of claim 16, wherein the green alga is *Haematococcus pluvialis*.

**18.** A method for producing a culture suspension of a green alga containing astaxanthin at a concentration of 150 mg or more per 1 L of the culture suspension, comprising:

cultivating an encysted green alga that can produce astaxanthin in a nutrient medium with a supply of carbon dioxide under irradiation with light at a photosynthetically active photon flux input of 25000 $\mu$mol-photon/m$^3$/s or more.

**19.** The method of claim 18, wherein the nutrient medium is an autotrophic medium.

**20.** The method of claim 18 or 19, wherein the photosynthetically active photon flux input is not more than 750000 $\mu$mol-photon/m$^3$/s.

**21.** The method of any of claims 18-20, wherein the supply of carbon dioxide is provided by bubbling a gas containing carbon dioxide at a concentration of 1 to 3 v/v% at a rate of 0.2 to 2 v/v/min.

**22.** A culture suspension of a green alga that can produce astaxanthin, which contains astaxanthin at a concentration of 150 mg or more per 1 L of the culture suspension and in which the green alga contains astaxanthin in an amount of 700 pg/cell or more.

**23.** The culture suspension of the green alga of claim 22, wherein the green alga is a unicellular alga belonging to the genus *Haematococcus*.

**24.** The culture suspension of the green alga of claim 23, wherein the green alga is *Haematococcus pluvialis*.

**25.** A method for producing astaxanthin, comprising:

cultivating an encysted green alga that can produce astaxanthin in a nutrient medium with a supply of carbon dioxide under irradiation with light at a photosynthetically active photon flux input of 25000 $\mu$mol-photon/m$^3$/s or more;
drying the resultant green alga; and
extracting astaxanthin from the resultant dry product of the green alga.

**26.** The method of claim 25, wherein the supply of carbon dioxide is provided by bubbling a gas containing carbon dioxide at a concentration of 1 to 3 v/v% at a rate of 0.2 to 2 v/v/min.

**Patentansprüche**

**1.** Verfahren zur Herstellung von Grünalgen umfassend die Kultivierung einer zystenbildenden Grünalge, welche Astaxanthin produzieren kann, in einem Nährmedium mit einer Versorgung mit Kohlendioxid und unter Bestrahlung mit Licht bei einem photosynthetisch aktivem Photon-Fluss-Input von 25000 $\mu$mol-photon/m$^3$ /s oder höher.

**2.** Verfahren nach Anspruch 1, worin das Nährmedium ein autotrophes Medium ist.

**3.** Verfahren nach einem der Ansprüche 1 oder 2, worin der photosynthetisch aktive Photon-Fluss-Input nicht mehr

als 750000 $\mu$mol-photon/m$^3$ /s ist.

4. Verfahren nach einem der Ansprüche 1 bis 3, worin die Grünalge Astaxanthin in einer Konzentration von mindestens 1.2 wt% des Nassanteils der Algen enthält.

5. Verfahren nach einem der Ansprüche 1 bis 4, worin die Versorgung mit Kohlendioxid durch Einblasen eines Gases mit einer Kohlendioxidkonzentration von 1 bis 3 v/v% bei einem Fluss von 0.2 bis 2 v/v/min erbracht wird.

6. Grünalge, welche Astaxanthin produzieren kann, worin Astaxanthin in einer Menge von 700 pg/Zelle oder mehr enthalten ist.

7. Grünalge nach Anspruch 6, wobei sie Astaxanthin in einer Konzentration von mindestens 1.2 wt% des Nassanteils der Algen enthält.

8. Grünalge nach einem der Ansprüche 6 oder 7, wobei sie eine einzellige Alge der Gattung *Haematococcus* ist.

9. Grünalge nach Anspruch 8, wobei sie *Haematococcus pluvialis* ist.

10. Verfahren zur Herstellung eines Trockenproduktes aus Grünalgen umfassend:

   die Kultivierung einer zystenbildenden Grünalge, welche Astaxanthin produzieren kann, in einem Nährmedium mit einer Versorgung mit Kohlendioxid und unter Bestrahlung mit Licht bei einem photosynthetisch aktivem Photon-Fluss-Input von 25000 vmol-photon/m$^3$ /s oder höher; und
   das Trocknen der resultierenden Grünalge.

11. Verfahren nach Anspruch 10, worin das Nährmedium ein autotrophes Medium ist.

12. Verfahren nach einem der Ansprüche 10 oder 11, worin der photosynthetisch aktive Photon-Fluss-Input nicht mehr als 750000 $\mu$mol-photon/m$^3$ /s ist.

13. Verfahren nach einem der Ansprüche 10 bis 12, worin das Trockenprodukt Astaxanthin in einer Konzentration von mindestens 5 wt% enthält.

14. Verfahren nach einem der Ansprüche 10 bis 13, worin die Versorgung mit Kohlendioxid durch Einblasen eines Gases mit einer Kohlendioxidkonzentration von 1 bis 3 v/v% bei einem Fluss von 0.2 bis 2 v/v/min erbracht wird.

15. Trockenprodukt aus Grünalgen, welche Astaxanthin produzieren können, worin Astaxanthin in einer Konzentration von mindestens 5 wt% und einer Menge von mindestens 700 pg/Zelle enthalten ist.

16. Trockenprodukt nach Anspruch 15, worin die Grünalge eine einzellige Alge der Gattung *Haematococcus* ist.

17. Trockenprodukt nach Anspruch 16, worin die Grünalge *Haematococcus pluvialis* ist.

18. Verfahren zur Herstellung einer Kultursuspension aus Grünalgen enthaltend Astaxanthin in einer Konzentration von mindestens 150 mg pro Liter Kultursuspension umfassend die Kultivierung einer zystenbildenden Grünalge, welche Astaxanthin produzieren kann, in einem Nährmedium mit einer Versorgung mit Kohlendioxid und unter Bestrahlung mit Licht bei einem photosynthetisch aktivem Photon-Fluss-Input von 25000 $\mu$mol-photon/m$^3$ /s oder höher.

19. Verfahren nach Anspruch 18, worin das Nährmedium ein autotrophes Medium ist.

20. Verfahren nach einem der Ansprüche 18 oder 19, worin der photosynthetisch aktive Photon-Fluss-Input nicht mehr als 750000 $\mu$mol-photon/m$^3$ /s ist.

21. Verfahren nach einem der Ansprüche 18 bis 20, worin die Versorgung mit Kohlendioxid durch Einblasen eines Gases mit einer Kohlendioxidkonzentration von 1 bis 3 v/v% bei einem Fluss von 0.2 bis 2 v/v/min erbracht wird.

22. Kultursuspension aus Grünalgen, welche Astaxanthin produzieren können, worin Astaxanthin in einer Konzentration von mindestens 150 mg pro Liter Kultursuspension enthalten ist, und worin die Grünalgen Astaxantin in einer Menge von mindestens 700 pg/Zelle enthalten.

23. Kultursuspension aus Grünalgen nach Anspruch 22, worin die Grünalge eine einzellige Alge der Gattung *Haematococcus* ist.

24. Kultursuspension aus Grünalgen nach Anspruch 23, worin die Grünalge *Haematococcus pluvialis* ist.

25. Verfahren zur Herstellung von Astaxanthin umfassend:

Kultivierung einer zystenbildenden Grünalge, welche Astaxanthin produzieren kann, in einem Nährmedium mit einer Versorgung mit Kohlendioxid und unter Bestrahlung mit Licht bei einem photosynthetisch aktivem Photon-Fluss-Input von 25000 $\mu$mol-photon/m$^3$ /s oder höher;
Trocknen der resultierenden Grünalge; und
Extrahieren von Astaxanthin aus dem resultierenden Trockenprodukt aus Grünalgen.

26. Verfahren nach Anspruch 25, worin die Versorgung mit Kohlendioxid durch Einblasen eines Gases mit einer Kohlendioxidkonzentration von 1 bis 3 v/v% bei einem Fluss von 0.2 bis 2 v/v/min erbracht wird.

**Revendications**

1. Procédé pour produire une algue verte comprenant la culture d'une algue verte enkystée qui peut produire de l'astaxanthine dans un milieu nutritif avec un apport de dioxyde de carbone sous irradiation avec de la lumière à un flux entrant de photons actifs du point de vue de la photosynthèse de 25000 $\mu$mol-photons/m$^3$/s ou plus.

2. Procédé selon la revendication 1 où le milieu nutritif est un milieu autotrophe.

3. Procédé selon la revendication 1 ou 2 où le flux entrant de photons actifs du point de vue de la photosynthèse n'est pas supérieur à 750000 $\mu$mol-photons/m$^3$/s.

4. Procédé selon l'une quelconque des revendications 1-3 où l'algue verte contient de l'astaxanthine à une concentration de 1,2 % en masse ou plus par cellule d'algue humide.

5. Procédé selon l'une quelconque des revendications 1-4 où l'apport de dioxyde de carbone est assuré par barbotage d'un gaz contenant du dioxyde de carbone à une concentration de 1 à 3 % v/v à un débit de 0,2 à 2 v/v/min.

6. Algue verte qui peut produire de l'astaxanthine, qui contient de l'astaxanthine en une quantité de 700 pg/cellule ou plus.

7. Algue verte selon la revendication 6 qui contient de l' astaxanthine à une concentration de 1,2 % en masse ou plus par cellule d'algue humide.

8. Algue verte selon la revendication 6 ou 7 qui est une algue unicellulaire appartenant au genre *Haematococcus.*

9. Algue verte selon la revendication 8 qui est *Haematococcus pluvialis*.

10. Procédé pour produire un produit sec d'une algue verte comprenant :

la culture d'une algue verte enkystée qui peut produire de l'astaxanthine dans un milieu nutritif avec un apport de dioxyde de carbone sous irradiation avec de la lumière à un flux entrant de photons actifs du point de vue de la photosynthèse de 25000 $\mu$mol-photons/m$^3$/s ou plus ; et
le séchage de l'algue verte résultante.

11. Procédé selon la revendication 10 où le milieu nutritif est un milieu autotrophe.

12. Procédé selon la revendication 10 ou 11 où le flux entrant de photons actifs du point de vue de la photosynthèse n'est pas supérieur à 750000 $\mu$mol-photons/m$^3$/s.

**13.** Procédé selon la revendication 10, 11 ou 12 où le produit sec contient de l'astaxanthine à une concentration de 5 % en masse ou plus.

**14.** Procédé selon l'une quelconque des revendications 10-13 où l'apport de dioxyde de carbone est assuré par barbotage d'un gaz contenant du dioxyde de carbone à une concentration de 1 à 3 % v/v à un débit de 0,2 à 2 v/v/min.

**15.** Produit sec d'une algue verte qui peut produire de l'astaxanthine, qui contient de l'astaxanthine à une concentration de 5 % en masse ou plus et contient de l'astaxanthine en une quantité de 700 pg/cellule ou plus.

**16.** Produit sec d'une algue verte selon la revendication 15 où l'algue verte est une algue unicellulaire appartenant au genre *Haematococcus*.

**17.** Produit sec d'une algue verte selon la revendication 16 où l'algue verte est *Haematococcus pluvialis*.

**18.** Procédé pour produire une suspension de culture d'une algue verte contenant de l'astaxanthine à une concentration de 150 mg ou plus par L de suspension de culture, comprenant :

la culture d'une algue verte enkystée qui peut produire de l'astaxanthine dans un milieu nutritif avec un apport de dioxyde de carbone sous irradiation avec de la lumière à un flux entrant de photons actifs du point de vue de la photosynthèse de 25000 $\mu$mol-photons/m$^3$/s ou plus.

**19.** Procédé selon la revendication 18 où le milieu nutritif est un milieu autotrophe.

**20.** Procédé selon la revendication 18 ou 19 où le flux entrant de photons actifs du point de vue de la photosynthèse n'est pas supérieur à 750000 $\mu$mol-photons/m$^3$/s.

**21.** Procédé selon l'une quelconque des revendications 18-20 où l'apport de dioxyde de carbone est assuré par barbotage d'un gaz contenant du dioxyde de carbone à une concentration de 1 à 3 % v/v à un débit de 0,2 à 2 v/v/min.

**22.** Suspension de culture d'une algue verte qui peut produire de l'astaxanthine, qui contient de l'astaxanthine à une concentration de 150 mg ou plus par L de suspension de culture et où l'algue verte contient de l'astaxanthine en une quantité de 700 pg/cellule ou plus.

**23.** Suspension de culture de l'algue verte selon la revendication 22 où l'algue verte est une algue unicellulaire appartenant au genre *Haematococcus*.

**24.** Suspension de culture de l'algue verte selon la revendication 23 où l'algue verte est *Haematococcus pluvialis*.

**25.** Procédé pour produire de l'astaxanthine comprenant :

la culture d'une algue verte enkystée qui peut produire de l'astaxanthine dans un milieu nutritif avec un apport de dioxyde de carbone sous irradiation avec de la lumière à un flux entrant de photons actifs du point de vue de la photosynthèse de 25000 $\mu$mol-photons/m$^3$/s ou plus ;
le séchage de l'algue verte résultante ; et
l'extraction de l'astaxanthine du produit sec de l'algue verte résultant.

**26.** Procédé selon la revendication 25 où l'apport de dioxyde de carbone est assuré par barbotage d'un gaz contenant du dioxyde de carbone à une concentration de 1 à 3 % v/v à un débit de 0,2 à 2 v/v/min.

Fig. 1

Fig. 2

Fig. 3

Fig. 4

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8901977 A
- JP 1187082 A
- JP 3083577 A
- JP 2000060532 A
- JP 7039389 A
- JP 2004129504 A
- WO 2005116238 A

**Non-patent literature cited in the description**

- **FABREGAS et al.** *J. Biotech.,* 2001, vol. 89, 65-71
- **TJAHJONO et al.** *BIOTECHNOLOGY LETTERS,* 1994, vol. 16, 133-138
- **Kang et al.** Comparison of heterotrophic and photoautotrophic induction on astaxanthin production by Haematococcus pluvialis. *APPLIED MICROBIOLOGY AND BIOTECHNOLOGY,* 02 August 2005, vol. 68, 237-241
- **Lababpour et al.** Effects of nutrient supply methods and illumination with blue light emitting diodes (LEDs) on astaxanthin production by Haematococcus pluvialis. *JOURNAL OF BIOSCIENCE AND BIOENGINEERING,* 06 December 2004, vol. 98, 452-456
- **Katsuda.** Astaxanthin production by Haematococcus pluvialis under illumination with LEDs. *ENZYME AND MICROBIAL TECHNOLOGY,* 06 July 2004, vol. 35, 81-86
- Sorui Kenkyuho. Kyoritsu Shuppan, 1979